# EUROPEAN PATENT APPLICATION

(11) **EP 4 394 043 A1**
(43) Date of publication of application: **03.07.2024**
(21) Application number: 22860554.9
(22) Date of filing: 24.08.2022
(51) Int. Cl.: C12N 15/85, C12N 5/10, A61K 39/395

(54) **METHOD FOR MODIFYING CELL**

(30) Priority: 24.08.2021 CN 202110974813
(71) Applicant: Cells & Genes Biotech (Shanghai) Co., Ltd, Shanghai 201203 (CN)
(72) Inventor: LI, Linhong, Shanghai 201203 (CN); ZHU, Xiangyang, Shanghai 201203 (CN); LIU, Wuqing, Shanghai 201203 (CN); XIN, Pengcheng, Shanghai 201203 (CN); WANG, Li, Shanghai 201203 (CN); YU, Shijun, Shanghai 201203 (CN); YANG, Xingxing, Shanghai 201203 (CN)
(74) Representative: Santoro, Sofia
(86) International application number: PCT/CN2022/114588
(87) International publication number: WO 2023/025208

(57) **Abstract**

Provided is a method for modifying a cell, which comprises the following steps: transfecting a target molecule and a screening marker molecule to a cell in a non-viral manner, so that the cell expresses the target molecule and the screening marker molecule. Also provided is a modified cell obtained using the method. The method can effectively enrich the desired cell and increase cell positivity with respect to the target molecule.

## Description

### Technical Field

The present application relates to the field of biomedicine, and specifically to a method for modifying cells.

### Background of the Invention

Non-viral transfection has great potential advantages in the field of cell therapy. When using it to prepare engineered cell therapy products required for cell therapy (e.g., CAR-T cells), it is necessary to quickly obtain high cell viability, high positive rate, high cell function, and a large number of cells. To achieve this goal, various methods have been attempted. For example, increasing the number of initiating cells or extending the cultivation time can compensate for the reduction in cell proliferation capacity caused by non-viral transfection methods, resulting in insufficient cells in the final product. However, increasing the number of initiating cells has problems such as high cost, and prolonging the cultivation time will change cell characteristics, greatly reducing the *in vivo* function of cells. For instance, the electric field strength in physical transfection can be increased, or the concentration of transfection auxiliary molecules (lipids) and DNA can be increased in chemical transfection to improve the transfection positive rate. However, these methods often come with a decrease in cell viability, sustained proliferation capacity, and cell function, so they still cannot achieve therapeutic goals. Moreover, although the positive rate of the screened cells can be improved by using commercially available GMP-grade kits, this still cannot ensure that the screened cells have good cell viability and cell function simultaneously. Therefore, how to produce cell therapy products that meet the requirements of cell therapy using non-viral methods in a shorter period has long been a major issue faced by the industry. This series of issues has prevented non-viral transfection from being effectively applied to cell therapy.

### Summary of the Invention

The present application provides a special non-viral method for modifying cells, which can achieve higher transfection efficiency, higher cell viability, and higher cell proliferation rate for T cells transfected with non-viral transfection methods. On this basis, additional screening and enrichment methods are used to obtain cell products with a higher positive rate to meet the requirements of cell therapy. The method of the present application achieves the quality requirements of plasmids by defining and processing plasmids, and uses electroporation to introduce target molecules with biological functions (e.g., tumor-killing effects) and screening molecules that can participate in screening into specific sites of cellular DNA, so that the modified cells can co-express target molecules and screening marker molecules. Through screening and enrichment using screening molecules, the proportion of cells expressing target molecules can be further significantly increased, and this comprehensive step can be used to prepare cell therapy products. The method of the present application can obtain cells with high cell viability, high sustained proliferation capacity, high cell function, high positive rate of target molecules, and unchanged characteristics after cryopreservation in a relatively short time (e.g., about 3-9 days). The method may be used to prepare CAR-T cells. For example, the method may be used to prepare CAR-T cells that express a high proportion of proteins with TCM memory cell characteristics (CCR7⁺ or CD62L⁺).

In one aspect, the present application provides a method for modifying a cell, comprising the following step: introducing a target molecule and/or a screening marker molecule into a cell via non-viral transfection, so that the cell expresses the target molecules and the screening marker molecules.

In another aspect, the present application provides a method for increasing positive rate of target molecule transfection, comprising the following step: introducing the target molecule and/or screening marker molecule into a cell via non-viral transfection, so that the cell expresses the target molecule and the screening marker molecule.

In some embodiments, the cell simultaneously expresses the target molecule and the screening marker molecule.

In some embodiments, the transfection comprises transient transfection.

In some embodiments, the transfection comprises electroporation.

In some embodiments, the transfection comprises stable transfection.

In some embodiments, the target molecule and/or the screening marker molecule are integrated into cell genome.

In some embodiments, the transfection comprises using transposon systems and/or using gene editing method for knock-in.

In some embodiments, the gene editing method is selected from one or more of the following group: CRISPR/Cas systems, RNA editing systems ADAR, RNA-guided endonucleases, zinc finger nucleases, Mega-TAL nucleases, TALENs, and Meganucleases.

In some embodiments, the non-viral transfection uses linear or circular nucleic acid molecule having an encoding region.

In some embodiments, the circular nucleic acid molecule comprises a circular plasmid or a supercoiled plasmid.

In some embodiments, the plasmid comprises nucleic acid molecules encoding the target molecule and/or nucleic acid molecule encoding the screening marker molecule.

In some embodiments, the plasmid serves as a donor plasmid in a knock-in process via gene editing.

In some embodiments, the nucleic acid molecule comprises ssDNA and/or dsDNA.

In some embodiments, the nucleic acid molecule comprises homology arms.

In some embodiments, the plasmid comprises plasmids produced by bacteria.

In some embodiments, the plasmid comprises nanoplasmids and/or minicircle DNA vectors.

In some embodiments, in a transfection mixture comprising the plasmid, the content of nucleic acid molecule or fragment thereof derived from a microbial genome is less than about 10% of the total content of nucleic acid molecules in the transfection mixture.

In some embodiments, in a transfection mixture comprising the plasmid, the content of nucleic acid molecule or fragment thereof derived from microbial genomes is less than about 2% of the total content of nucleic acid molecules in the transfection mixture.

In some embodiments, in the transfection mixture comprising the plasmid, the content of nucleic acid molecule or fragment thereof derived from a microbial genome is less than about 5‰ of the total content of nucleic acid molecules in the transfection mixture.

In some embodiments, in the transfection mixture comprising the plasmid, the content of nucleic acid molecule or fragment thereof derived from a microbial genome is less than about 1 %o of the total content of nucleic acid molecules in the transfection mixture.

In some embodiments, in the transfection mixture comprising the plasmid, the content of the nucleic acid molecule or fragment thereof with a size of at least about 48kb is less than about 10% of the total content of nucleic acid molecules in the transfection mixture.

In some embodiments, in the transfection mixture comprising the plasmid, the content of the nucleic acid molecule or fragment thereof with a size of at least about 48kb is less than about 2% of the total content of nucleic acid molecules in the transfection mixture.

In some embodiments, in the transfection mixture comprising the plasmid, the content of the nucleic acid molecule or fragment thereof with a size of at least about 48kb is less than about 5‰ of the total content of nucleic acid molecules in the transfection mixture.

In some embodiments, in the transfection mixture comprising the plasmid, the content of the nucleic acid molecule or fragment thereof with a size of at least about 48kb is less than about 1‰ of the total content of nucleic acid molecules in the transfection mixture.

In some embodiments, the size of the nucleic acid molecule or fragment thereof with a size of at least about 48kb is at least about 1Mb.

In some embodiments, the size of the nucleic acid molecule or fragment thereof with a size of at least about 48kb is at least about 10Mb.

In some embodiments, the nucleic acid molecule or fragment thereof with a size of at least about 48kb is derived from a microorganism.

In some embodiments, the microorganism is selected from one or more of the following group: bacteria, fungi, actinomycetes, mycoplasma, chlamydia, rickettsia, and spirochetes.

In some embodiments, the microorganism comprises Gram-negative bacteria.

In some embodiments, the microorganism comprises *Escherichia coli.*

In some embodiments, the method comprises the step of treating the plasmid with deoxyribonuclease (DNase).

In some embodiments, the DNase is capable of non-specifically cleaving linear DNA.

In some embodiments, the DNase is a nucleic acid exonuclease.

In some embodiments, the treatment comprises contacting the transfection mixture with the DNase in the presence of Mg²⁺ and Ca²⁺.

In some embodiments, after the treatment by the DNase, in the transfection mixture comprising the plasmid, the content of nucleic acid molecule or fragment thereof derived from a microbial genome is less than about 10% of the total content of nucleic acid molecules in the transfection mixture.

In some embodiments, after the treatment by the DNase, in the transfection mixture comprising the plasmid, the content of the nucleic acid molecule or fragment thereof with a size of at least about 48kb is less than about 10% of the total content of nucleic acid molecules in the transfection mixture.

In some embodiments, the cell comprises eukaryotic cell.

In some embodiments, the cell comprises immune cell, stem cell, fibrocyte, and/or muscle cell.

In some embodiments, the stem cell comprises pluripotent stem cell.

In some embodiments, the stem cell comprises hematopoietic stem cell and/or mesenchymal stem cell.

In some embodiments, the immune cells is selected from the group consisting of: unactivated or activated T lymphocyte, B lymphocytes, NK cell, macrophage, dendritic cell, monocyte, granulocyte, and mast cell.

In some embodiments, the immune cell comprises primary T cell.

In some embodiments, the target molecule comprises antibody or antigen-binding fragment, chimeric antigen receptor (CAR), cytokine, and/or chemokine.

In some embodiments, the chimeric antigen receptor targets one or more targets.

In some embodiments, the chimeric antigen receptor targets CD19 and CD20.

In some embodiments, the chimeric antigen receptor targets CD 19 and CD22.

In some embodiments, the antibody or antigen-binding fragment comprises bispecific antibody or antigen-binding fragment.

In some embodiments, the antibody or antigen-binding fragment comprises bispecific T cell engager (BiTE).

In some embodiments, the screening marker molecule comprises a structural domain capable of binding to a screening material.

In some embodiments, the screening material comprises magnetic bead.

In some embodiments, the screening marker molecule comprises CD34 and/or EGFRT variant.

In some embodiments, the type of the screening marker molecule corresponds to the type of the cell and/or the type of the target molecule.

In some embodiments, the cell is an immune cell, and the screening marker molecule is CD34 and/or EGFRT variant.

In some embodiments, the method comprises the following step: contacting the cell expressing the target molecule and the screening marker molecule with the screening material.

In some embodiments, the method further comprises the following step: collecting the cell bound to the screening material.

In some embodiments, the method further comprises the following step: separating the cell bound to the screening material from the screening material.

In another aspect, the present application provides a cell and/or a cell line prepared using the method of the present application.

In another aspect, the present application provides a modified immune cell simultaneously expressing the target molecule and the screening marker molecule.

In some embodiments, the immune cell is selected from the group consisting of: unactivated or activated T lymphocytes, B lymphocytes, NK cells, macrophages, dendritic cells, monocytes, granulocytes, and mast cells.

In some embodiments, the target molecule comprises an antibody or antigen-binding fragment, chimeric antigen receptor (CAR), cytokine, and/or chemokine.

In some embodiments, the chimeric antigen receptor targets one or more targets.

In some embodiments, the chimeric antigen receptor targets CD 19 and CD20.

In some embodiments, the chimeric antigen receptor targets CD 19 and CD22.

In some embodiments, the antibody or antigen-binding fragment comprises bispecific antibody or antigen-binding fragment.

In some embodiments, the antibody or antigen-binding fragment comprises bispecific T-cell engager (BiTE).

In some embodiments, the screening marker molecule comprises structural domain capable of binding to the screening material.

In some embodiments, the screening material comprises magnetic bead.

In some embodiments, the screening marker molecule comprises CD34 and/or EGFRT variant.

In some embodiments, the type of the screening marker molecule is selected based on the type of the cell and/or the target molecule.

In some embodiments, the cell is immune cell, and the screening marker molecule is CD34 and/or EGFRT variant.

Those skilled in the art can easily discern other aspects and advantages of the present application from the detailed description below. The detailed description only shows and describes exemplary embodiments of the present application. As those skilled in the art will appreciate, the disclosure of the present application enables those skilled in the art to modify the specific embodiments disclosed without departing from the spirit and scope of the inventions involved in the present application. Accordingly, the descriptions in the drawings and specifications of the present application are merely illustrative and not limiting.

### Brief Description of the Drawings

The specific features of the invention involved in the present application are as shown in the accompanying claims. A better understanding of the features and advantages of the invention involved in the present application can be obtained by referring to the detailed description of the exemplary embodiments and the drawings below. A brief description of the drawings is as follows:
FIG. 1 shows the cell viability of the modified cells of the present application.
FIG. 2 shows the cell proliferation results of the modified cells of the present application.
FIG. 3 shows the expression of proteins in the modified cells of the present application.
FIG. 4 shows the expression of proteins in the modified cells of the present application after enrichment.
FIG. 5 shows the cell proliferation results of the modified cells of the present application after enrichment.
FIGs. 6A-6B show the positive rate of protein expression by the modified cells of the present application after enrichment.
FIG. 7 shows the cell viability of the modified cells of the present application after enrichment followed by cryopreservation and recovery.
FIG. 8 shows the cell count of the modified cells of the present application after enrichment followed by cryopreservation and recovery.
FIG. 9 shows the proliferation results of the modified cells of the present application after enrichment followed by cryopreservation, recovery, and activation by target cells.
FIG. 10 shows the killing of *in vitro* target cells by the modified cells of the present application after enrichment.
FIG. 11 shows the killing of *in vivo* tumors by the modified cells of the present application after enrichment.
FIG. 12 shows the expression of proteins by the modified cells of the present application after enrichment.
FIG. 13 shows the killing of *in vitro* target cells by the modified cells of the present application after enrichment.
FIG. 14 shows the killing of *in vivo* tumors by the modified cells of the present application after enrichment.
FIG. 15 shows the properties of plasmids containing nucleic acid molecules derived from microbial genomes with different contents.

### Description

The following specific embodiments illustrate the implementation of the present invention, and those skilled in the art can easily understand other advantages and effects of the present invention from the content disclosed in the present specification.

### Definitions

In the present application, the term "stable transfection" generally refers to that exogenous nucleic acid molecules are introduced and intergrated into the genome of the transfected cells. For example, the exogenous gene is intergrated into the genome of the transfected cells.

In the present application, the term "transient transfection" generally refers to a transfection method in which the exogenous gene transfected into the cell is not integrated into the genome of the cell. Transient transfection can achieve rapid expression of exogenous genes in a short period (e.g., at least about 1 day, at least about 2 days). The exogenous gene transfected into the cell by transient transfection may be gradually lost as the cell grows and/or divides. The transient transfection may have advantages selected from the following group: easy operation, short experimental cycle, high expression efficiency, safety, and no need for gene screening. The operation steps of the transient transfection can be known to those skilled in the art. For example, the transient transfection can be achieved via liposome mediated transfection. For example, the transient transfection may comprise electroporation transfection (e.g., electroporation). The transient transfection can use transfection reagents, such as FuGENE6.

In the present application, the term "deoxyribonuclease (DNase)" generally refers to an enzyme capable of cleaving the phosphodiester bonds on the DNA backbone. The DNase may be a type of nucleases. The DNase may digest double-stranded DNA into deoxynucleotides (e.g., under weakly alkaline conditions). For example, the DNase may not have activity (e.g., cannot cleave) on circular or superhelical nucleic acid molecule or fragment thereof (e.g., closed circular double-stranded DNA and/or superhelical DNA). DNase may also have some activity on circular or linear single-stranded DNA nucleic acid molecule or fragment thereof, but the activity may be lower (e.g., lower efficiency in cleavage). DNase may be a DNA exonuclease. DNase may be an ATP-Dependent DNase, such as Plasmid-Safe^{™} ATP-Dependent DNase.

In the present application, the term "transfection efficiency" generally refers to the relative amount of materials introduced into and/or expressed by the transfected cells. In the present application, the transfection may refer to the introduction of one or more materials (e.g., polynucleotides) into cells. The introduced material may be stably or transiently maintained in the transfected cells. In the present application, the transfection efficiency may be measured by the amount of introduced materials.

In the present application, the term "nucleic acid molecule or its fragments" generally refers to nucleotides (e.g., ribonucleotides, deoxyribonucleotides, and/or modified forms of the foregoing) or fragments thereof. The nucleic acid molecule or fragment thereof may comprise the form of a polymer of nucleotides or fragment thereof. In some cases, the nucleic acid molecule or fragment thereof may be interchangeable with "polynucleotides." The nucleic acid molecule may comprise DNA, RNA, cDNA, sense and antisense strands of genomic DNA, and synthetic forms, mixtures, and/or polymers thereof. The nucleic acid molecule or fragment thereof may include any topological conformation, such as single-stranded, double-stranded, partially double-stranded, triple-stranded, hairpin structures, circular, and/or catenated conformations. Nucleotides in the nucleic acid molecule or fragment thereof may be natural or modified. Nucleotides in the nucleic acid molecule or fragment thereof may be linked together by naturally occurring and/or non-naturally occurring nucleotide bonds.

In the present application, the term "transfection mixture" generally refers to a mixture required for transfection. In the present application, the transfection mixture may comprise one or more materials to be introduced (e.g., polynucleotides). For example, the transfection mixture may comprise a nucleic acid molecule encoding an exogenous gene to be transfected. The transfection mixture may comprise a vector containing the nucleic acid molecule. For example, the transfection mixture may also comprise impurities (in some cases, the impurities may comprise nucleic acid molecule or fragment thereof). In the present application, the impurities may comprise nucleic acid molecule or fragment thereof derived from microorganisms. The impurities carried by the vector may include impurities carried by the vector (e.g., nucleic acid molecule or fragment thereof homologous or heterologous to the vector backbone).

In the present application, the term " total content of nucleic acid molecules" generally refers to the total mass of all substances with nucleotides in the transfection mixture. For example, the substances with nucleotides may comprise the nucleic acid molecule or fragment thereof and the materials.

In the present application, the term "microorganism" generally refers to eukaryotic and prokaryotic microbial species from the *Archaea, Bacteria*, and/or *Eucarya* domains. For example, the microorganisms may comprise bacteria, viruses, fungi, actinomycetes, rickettsiae, mycoplasmas, chlamydiae, and/or spirochetes. In the present application, the term bacteria generally refers to any type of prokaryote, including prokaryotes from all phyla within the prokaryote domain. The bacteria may comprise cocci, bacilli, spirilla, protoplasts, spheroplasts. The bacteria may comprise Gram-positive and Gram-negative bacteria. "Gram-negative" and "Gram-positive" refer to staining patterns using the Gram stain method, which is well known in the art (see, for example, Finegold and Martin, Diagnostic Microbiology, 6th edition, CV Mosby St. Louis, pages 13-15 (1982)).

In the present application, the term "nucleic acid molecule or fragment thereof derived from a microbial genome " generally refers to a nucleic acid molecule or fragments thereof, which are derived from the genome of the microorganism. Information about the genomes of microorganisms can be found in A genomic catalog of Earth's microbiomes, Nature Biotechnology (2020).

In the present application, the term "Gram-negative bacteria" generally refers to bacteria that do not retain the primary dye used in Gram staining but are stained by the counterstain. Therefore, Gram-negative bacteria usually appear red in the Gram staining method. The cell wall of the Gram-negative bacteria has a lower content of peptidoglycan and a higher content of lipids. For example, the cell wall of the Gram-negative bacteria may have a lipopolysaccharide layer. For example, the Gram-negative bacteria may comprise *Escherichia coli, Pseudomonas aeruginosa, Proteus species, Shigella species, Klebsiella pneumoniae, Brucella species, Haemophilus influenzae, Haemophilus parainfluenzae, Moraxella catarrhalis, Acinetobacter species, Yersinia species, Legionella pneumophila, Bordetella pertussis, Bordetella parapertussis, Shigella species, Pasteurella species, Vibrio cholerae, Bacillus parahaemolyticus,* and/or *Plesimonas shigelloides.*

In the present application, the term *"Escherichia coli"* generally refers to *Escherichia coli. Escherichia coli* belongs to the *Enterobacteriaceae* family, *Escherichia* genus.

In the present application, the term "immune cell" generally refers to cells that play a role in immune responses. The immune cell may comprise lymphocytes, monocytes, and/or granulocytes, as well as their precursors and/or mature derivatives. The immune cell may comprise T cells, B cells, Th cells, natural killer cells, monocytes, macrophages, eosinophils, basophils, mast cells, dendritic cells, and/or granulocytes. The immune cell may comprise immune effector cells. The immune effector cells may participate in immune responses, such as promoting immunoeffector responses. The immune effector cells may comprise T cells, for example, α/β T cells and γ/δ T cells, B cells, natural killer (NK) cells, natural killer T (NKT) cells, mast cells, and bone marrow-derived phagocytes.

In the present application, the term "stem cell" generally refers to a class of undifferentiated cells that have the ability to self-renew and retain varying degrees of potential to form differentiated cells and tissues. The stem cell may be impotent, multipotent, or totipotent. Impotent stem cells are derived stem cells that have lost their differentiation ability. Totipotent stem cells can form all cells and tissues found in a complete organism. For example, totipotent stem cells can form a complete organism.

In the present application, the term "multipotent stem cell" generally refers to stem cells that have the ability to form cells and tissues that are ultimately found in a complete organism but cannot form a complete organism. For example, the multipotent stem cells may proliferate for an extended period or virtually indefinitely in vitro while maintaining their undifferentiated state and displaying a normal karyotype (chromosomes). The multipotent stem cells may have the ability to differentiate into all three germ layers (ectoderm, mesoderm, and endoderm) under appropriate conditions. For example, the multipotent stem cells may comprise embryonic stem (ES) cells isolated from early embryos and/or isogenic EG cells isolated from primordial germ cells of fetal tissues.

In the present application, the term "mesenchymal stem cell" generally refers to cells that can produce mesenchymal lineages. The mesenchymal stem cells can be considered as part of multipotent stem cells. The mesenchymal stem cells can produce one or more mesenchymal lineage cells. The mesenchymal lineage cells can be derived from various tissues, for example, bone marrow tissue, adipose tissue, muscle tissue, reproductive tissue (e.g., amniotic, amniotic fluid, or umbilical cord tissue), skin tissue, bone tissue, and/or dental tissue.

In the present application, the term "immune cell" generally refers to cells that function in the immune response. The immune cells may comprise lymphocytes, monocytes, and/or granulocytes, as well as their precursors and/or mature derivatives. The immune cells may comprise T cells, B cells, Th cells, natural killer cells, monocytes, macrophages, eosinophils, basophils, mast cells, dendritic cells, and/or granulocytes. The immune cells may comprise immune effector cells. The immune effector cells may participate in immune responses, such as promoting immunoeffector responses. The immune effector cells may comprise T cells, for example, α/β T cells and γ/δ T cells, B cells, natural killer (NK) cells, natural killer T (NTK) cells, mast cells, and bone marrow-derived phagocytes.

In the present application, the term "fibrocyte" generally refers to functionally inactive fibroblasts. The fibrocytes may transform into fibroblasts (for example, they may participate in the repair process when tissue is damaged). The fibroblasts (also referred to as fibroblastic cells) may secrete structural proteins that constitute the extracellular matrix. The fibroblasts may be closely related to physiological or pathological processes such as wound healing and fibrosis.

In the present application, the term "muscle cell" generally refers to one cell or a group of cells derived from muscle. The muscle cells may be derived from cells and tissues of skeletal muscle, smooth muscle (for example, from the digestive tract, bladder, and blood vessels), and cardiac muscle. The muscle cells may comprise *in vitro* and *in vivo* myocytes. The muscle cells may also comprise differentiated and undifferentiated muscle cells, such as myocytes (e.g., myotubes), dividing and differentiating myoblasts, cardiomyocytes, and cardiomyocyte-derived myocytes and muscle tissues.

In the present application, the term "plasmid" generally refers to a construct containing genetic material. The plasmid may be designed to deliver genetic material (e.g., one or more nucleic acid sequences) into cells. The plasmid may contain autonomously replicating sequences of single-stranded or double-stranded nucleic acids (e.g., DNA or RNA) from any source. The term "plasmid" may be used interchangeably with the term "vector" in the present application. The plasmid may have different conformations, such as linear plasmids, circular plasmids, or supercoiled plasmids. The linear plasmid may be a linear DNA molecule. The supercoiled plasmid may comprise two nucleic acid strands that maintain intact structures (e.g., covalently closed circular DNA, cccDNA), and exhibit a supercoiled conformation. The circular plasmid may have at least one nucleic acid strand maintaining a complete circular structure. The plasmid may be not integrated into the cell genome.

In the present application, the term "multiple cloning site" or "MCS" generally refers to a nucleic acid sequence containing at least one restriction site. The multiple cloning site may link the nucleic acid molecule to the vector of the present application, for example, through the restriction site to achieve insertion of the nucleic acid molecule at a specified position. The restriction site may be a restriction endonuclease recognition site. For example, the restriction endonuclease may be AclU, HindIII, SspI, MLuCI, Tsp509I, PciI, AgeK, BspMI, BfuAI, SexAI, MLuI, BceAI, HpyCH4IV, HpyCH4III, BaeI, BsaXI, SpeI, BsrI, BmrI, BglII, AfeI, AluI, StuI, Seal, Clal, BspDI, PI-SceI, Nsil, AseI, SwaI, CspCI, MfeI, BssSI, BmgBI, PmLI, DraIII, AleI, EcoP15I, PvuII, AlwNI, or BtsCI-MutI.

In the present application, the term "antibody" generally refers to an immunoglobulin that is reactive to a specific protein, peptide, or fragments thereof. Antibodies may be from any class, including but not limited to IgG, IgA, IgM, IgD, and IgE, and antibodies from any subclass (e.g., IgG1, IgG2, IgG3, and IgG4). Antibodies may have heavy chain constant regions selected from, for example, IgG1, IgG2, IgG3, or IgG4. Antibodies may also have light chains selected from, for example, kappa (κ) or lambda (λ). The antibodies of the present application may be derived from any species.

In the present application, the term "antigen-binding fragment" generally refers to a portion of an antibody molecule that comprises amino acid residues that interact with the antigen and confer specificity and affinity of the antibody to the antigen. Examples of antigen-binding fragments may comprise, but are not limited to, Fab, Fab', F(ab)₂, Fv, F(ab')₂, scFv, di-scFv and/or dAb. In the present application, the term "Fab" generally refers to a fragment containing the heavy chain variable domain and the light chain variable domain, as well as the light chain constant domain and the first constant domain of the heavy chain (CH1); the term "Fab‴ generally refers to a fragment that differs from Fab by the addition of a small number of residues (including one or more cysteines from the antibody hinge region) at the carboxy terminus of the heavy chain CH1 domain; the term "F(ab')₂" generally refers to a dimer of Fab', an antibody fragment containing two Fab fragments connected via disulfide bridges in the hinge region. The term "Fv" generally refers to the minimal antibody fragment containing the complete antigen recognition and binding site. In some cases, this fragment may consist of a tight non-covalent dimer composed of a heavy chain variable region and a light chain variable region; the term "dsFv" generally refers to a disulfide-stabilized Fv fragment, in which the bond between a single light chain variable region and a single heavy chain variable region is a disulfide bond. The term "dAb fragment" generally refers to an antibody fragment composed of a VH domain. In the present application, the term "scFv" generally refers to a monovalent molecule in which one heavy chain variable domain and one light chain variable domain of an antibody are covalently linked together via a flexible peptide linker; such scFv molecules may have the general structure: NH₂-VL-linker-VH-COOH or NH₂-VH-linker-VL-COOH.

In the present application, the term "chimeric antigen receptor" (CAR) generally refers to a fusion protein that comprises an extracellular domain capable of binding to an antigen and at least one intracellular domain. CAR is the core component of chimeric antigen receptor T cells (CAR-T), which may comprise an antigen-binding domain (e.g., tumor-specific antigen and/or tumor-associated antigen), a transmembrane domain, a co-stimulatory domain, and an intracellular signaling domain. In the present application, the CAR may be based on the antigen-specificity (e.g., CD19) of an antibody and the intracellular domain of a T cell receptor activation. Genetically modified T cells expressing CAR may specifically recognize and eliminate malignant cells expressing the target antigen. For descriptions of CAR and CAR-T cells, see for example Sadelain M, Brentjens R, Rivi "ere I. The basic principles of chimeric antigen receptor design. Cancer Discov. 2013;3(4):388-398; Turtle CJ, Hudecek M, Jensen MC, Riddell SR. Engineered T cells for anti-cancer therapy. Curr Opin Immunol. 2012;24(5):633-639; Dotti G, Gottschalk S, Savoldo B, Brenner MK. Design and development of therapies using chimeric antigen receptor-expressing T cells. Immunol Rev. 2014;257(1):107-126; and WO2013154760, WO2016014789.

In the present application, the term "bispecific antibody" generally refers to an antibody having a variable region capable of recognizing more than one epitope on one or more antigens. Bispecific antibodies comprise, but are not limited to, full-length antibodies, antibodies with two or more VL and VH structural domains, antibody fragments, such as Fab, Fv, dsFv, scFv, bispecific antibodies, and covalently or non-covalently linked antibody fragments. In some cases, the bispecific antibody may recognize two different epitopes on the same or different antigens. In some cases, the bispecific antibody may recognize two different antigens.

In the present application, the term "antigen-binding domain" generally refers to a structural domain capable of binding to a target antigen. Antigen-binding domains may comprise specific antigen-binding antibodies and antigen receptors or fragments thereof, antibodies or their antigen-binding fragments. Antigen-binding domains may be domains capable of binding to tumor-associated antigens, comprising but not limited to: CD19, CD20, CD22, CD123, CD33/IL3Ra, CD138, CD33, BCMA, CS1, C-Met, EGFRvIII, CEA, Her2, GD2, MAG3, GPC3, and NY-ESO-1.

In the present application, the term "BiTE" generally refers to bispecific T cell engagers. The BiTE may be a single polypeptide chain molecule having two antigen-binding domains, one of which binds to a T cell antigen and the second to an antigen present on the surface of a target cell (see WO 2005/061547; Baeuerle, P et al. (2008) "BiTE®: A New Class Of Antibodies That Recruit T Cells Drugs of the Future 33:137-147; or Bargou et al. (2008) "Tumor Regression in Cancer Patients by Very Low Doses of a T Cell-Engaging Antibody," Science 321:974-977).

In the present application, the term "homology arm" generally refers to a polynucleotide suitable for targeting an exogenous gene to be knocked into a donor plasmid to the genome by homologous recombination. Homologous recombination may refer to recombination occurring between sister chromatids or between DNA molecules on the same chromosome containing homologous sequences or within molecules. The homology arm may have two (e.g., it may have a 5' homology arm and/or a 3' homology arm). The homology arms may be located upstream and downstream of the exogenous gene to be knocked into the donor plasmid. In some cases, the targeting site in the genome for the exogenous gene to be knocked in may be broken due to the effect of nucleases. The homology arms may be identical or have at least 80% identity to the DNA sequences at both ends (i.e., 5' and/or 3' ends) of the break at the target site. For example, the homology arms may have at least about 80%, at least about 85%, at least about 90%, at least about 91%, at least about 92%, at least about 93%, at least about 94%, at least about 95%, at least about 96%, at least about 97%, at least about 98%, at least about 99%, or 100% identity to the DNA sequences at both ends of the break at the target site. In the present application, the donor plasmid may comprise a 5' homology arm and/or a 3' homology arm.

In the present application, the term "cell line" generally refers to a clonal population of cells capable of continuous division. For example, the cell line may have acquired the ability to proliferate indefinitely *in vitro.*

In the present application, the term "gene editing" generally refers to the manipulation of inserting, deleting, and/or replacing nucleic acids into the genome. The gene editing can be achieved through homology-directed repair (HDR), non-homologous end joining (NHEJ), or single-base changes. The gene editing can use gene editing tools familiar to those skilled in the art, such as the zinc finger nuclease system (ZFN), the TALEN system, and/or CRISPR technique.

In the present application, the term "transposon system" generally refers to a system comprising the ability to excise from a donor polynucleotide (e.g., plasmid) and integrate into a target (e.g., cellular genomic DNA). The polynucleotide may be integrated into the target by a transposase. The transposon system may comprise a transposase. The transposase may transpose and mediate the transposed functional nucleic acid-protein complex. The transposon system may comprise transposon ends. The transposon ends may be the nucleotide sequences necessary for forming a complex with the transposase or integrase. The transposon ends may be double-stranded DNA. In some cases, the transposon ends may form a functional nucleic acid-protein complex with the transposon in the transposition reaction.

In the present application, the term "nanoplasmid" generally refers to a nanoplasmid, i.e., one or more lipid-based nanocarriers, polymer nanoparticles, metal nanoparticles, surfactant-based emulsions, dendrimers, buckyballs, nanowires, virus-like particles, peptide or protein-based particles (e.g., albumin nanoparticles), and/or composite materials using nanomaterials (e.g., lipid-polymer nanoparticles) generated nanoparticles. In some cases, the size of the nanoplasmid may be equal to or less than about 100 nanometers.

In the present application, the term "minicircle DNA vector" generally refers to a product of site-specific recombination of a parental plasmid (PP). The minicircle DNA vector is a supercoiled DNA molecule. Under the effect of recombinase, the parental plasmid is transformed into two circular DNAs, one containing a large amount of bacterial backbone sequences, called miniplasmid (MP); the other is a eukaryotic expression cassette containing only the target gene, i.e., minicircle (MC). The minicircle DNA vector may comprise a eukaryotic expression box.

In the present application, the term "gene editing knock-in" generally refers to a genetic engineering process (e.g., also called knock-in), which involves one-to-one replacement of DNA sequence information at a genetic locus or insertion of sequence information not found within the endogenous locus. The gene editing knock-in can utilize homologous recombination. For example, using homologous recombination, an exogenous functional gene (a gene not originally present in the genome or an inactivated gene) is transferred into a cell and undergoes homologous recombination with homologous sequences in the genome, allowing it to be inserted into the genome and expressed in the cell. For example, the gene editing knock-in may at least partially replace the cellular genome with an exogenous gene. The gene editing knock-in can use CRISPR technique to achieve targeted "knock-in".

In the present application, the term "about" generally refers to a value or numeric range within 30%, 25%, 20%, 15%, 10%, 9%, 8%, 7%, 6%, 5%, 4%, 3%, 2%, 1%, 0.5%, or 0.05% of the specified value or range involved in the present application. In the present application, when the term "about" precedes the first value in a series of two or more values, it applies to each value in the series.

### Detailed Description of the Invention

In one aspect, the present application provides a method for modifying a cell, comprising the following steps: introducing a target molecule and/or a screening marker molecule into a cell by non-viral transfection, so that the cell expresses the target molecule and the screening marker molecule.

In another aspect, the present application provides a method for increasing the positive rate of target molecule transfection, comprising the following steps: introducing the target molecule and/or the screening marker molecule into a cell by non-viral transfection, so that the cell expresses the target molecule and the screening marker molecule.

In the present application, the cell may express both the target molecule and the screening marker molecule simultaneously.

In the present application, the transfection may comprise transient transfection. For example, the transfection may comprise electroporation. The electroporation may refer to cell electroporation or cell electroporation (electroporation). The electroporation may utilize the effect of a strong instantaneous electric field to allow charged substances entering a cell by passing through a cell membrane with a certain permeability. The electroporation may produce little cytotoxicity. The cytotoxicity produced by the electroporation is significantly reduced compared to chemical transfection methods and/or viral transfection methods. The electroporation can be applied to almost all kinds of eukaryotic cells. The electroporation can be used for transient or stable expression of exogenous proteins.

In the present application, the transfection may comprise stable transfection.

In the present application, the transfection (e.g., stable transfection) may comprise transfection using a transposon system (e.g., comprising a Sleeping Beauty transposon system).

In the present application, the target molecule and/or the screening marker molecule may be integrated into the genome of the cell.

In the present application, the transfection may comprise gene editing knock-in.

In the present application, the gene editing method may be selected from one or more of the following group: CRISPR/Cas system, RNA editing system ADAR, RNA-guided endonuclease, zinc finger nuclease, Mega-TAL nuclease, TALENs, and Meganucleases. In the present application, the gene editing method may use a variety of nucleases, as long as the nuclease can help integrate the target molecule and/or the screening marker molecule into the cell genome.

In the present application, the gene editing method can be known to those skilled in the art, as long as it can achieve the purpose of gene editing, and is not limited to a specific method. In the present application, the gene editing method may be selected from one or more of the following group: CRISPR/Cas system, RNA editing system ADAR, RNA-guided endonuclease, zinc finger nuclease, Mega-TAL nuclease, TALENs, and Meganucleases. For example, the gene editing method may employ the CRISPR/Cas system.

In the present application, the gene editing may comprise gene editing knockout and/or gene editing knock-in. For example, the gene editing may comprise gene editing knock-in. For example, CRISPR/Cas system may be used for the gene editing knock-in.

For example, the CRISPR-CAS system may comprise a class of clustered regularly interspaced short palindromic repeats (CRISPRs) and some functionally related proteins (CRISPR-associated, Cas). The Cas protein coding gene may comprise Cas9, Cas1, Cas2, and Csn2. For example, it may be Cas9. The CRISPR-CAS system (e.g., CRISPR/Cas9 system) may have targeted cleaving specificity for DNA molecules.

For example, the gene editing knock-in may be a process in which the Cas protein targets a specific DNA sequence in the cell to insert a DNA sequence. The gene editing knock-in may be a process mediated by the Cas protein (e.g., Cas9 protein) that causes homologous recombination of the exogenous gene to be knocked in the donor plasmid with the specific DNA sequence in the targeted cell.

In the present application, the non-viral transfection may use linear or circular nucleic acid molecules with encoding sequences. For example, the circular nucleic acid molecule may comprise circular plasmids or supercoiled plasmids.

In the present application, the plasmid may be a DNA plasmid. For example, the DNA plasmid may be a double-stranded, circular DNA molecule. For example, the DNA plasmid may be a double-stranded, linear DNA molecule. In the present application, the plasmid may be artificially synthesized.

In the present application, the plasmid may comprise plasmids produced by bacteria. In the present application, the plasmid may comprise nanoplasmids and/or minicircle DNA vectors.

In the present application, the plasmid may comprise multiple cloning sites. The plasmid may encode at least one target molecule. The target molecule may encode one or more antigen-binding fragments (e.g., antibodies), chimeric antigen receptors, cytokines, and/or chemokines. The target molecule may also encode any one or more functional proteins. For example, the functional protein may be used to treat diseases related to gene defects. For example, the functional protein may be a protein that is absent and/or mutated in the host of the transfected cell. For example, the exogenous gene expressing the functional protein may be transfected into fibrocytes (e.g., primary fibroblasts), muscle cells, and/or stem cells (e.g., iPS cells).

In the present application, the nucleic acid molecule encoding the target molecule and/or the nucleic acid molecule encoding the screening marker molecule may be expressed by endogenous promoters or exogenous promoters. For example, the plasmid may contain the exogenous promoter.

In the present application, the plasmid may comprise nucleic acid molecules encoding the target molecule and/or nucleic acid molecules encoding the screening marker molecule. For example, the nucleic acid molecule encoding the target molecule and the nucleic acid molecule encoding the screening marker molecule may be located on the same plasmid. For example, the nucleic acid molecule encoding the target molecule and/or the nucleic acid molecule encoding the screening marker molecule may be connected by linker sequences. The linker sequence does not affect the expression and/or function of the nucleic acid molecule encoding the target molecule and/or the nucleic acid molecule encoding the screening marker molecule. For example, the linker sequence may comprise a sequence encoding a self-cleaving peptide. For example, the linker sequence may comprise a nucleic acid molecule encoding the 2A sequence.

In the present application, the plasmid may serve as a donor plasmid in the gene editing knock-in. For example, the donor plasmid may be double-stranded DNA or single-stranded DNA. The donor plasmid may serve as a donor template for the HDR repair mechanism.

In the present application, the nucleic acid molecule may comprise ssDNA and/or dsDNA. In the present application, the nucleic acid molecule may be artificially synthesized.

In the present application, the plasmid may comprise ssDNA and/or dsDNA. In the present application, the plasmid (e.g., the donor plasmid) may comprise homology arms. The plasmid may be artificially synthesized.

In the present application, in the transfection mixture comprising the plasmid, the content of nucleic acid molecule or fragment thereof derived from the microbial genome may be less than about 10% of the total content of nucleic acid molecules in the transfection mixture (for example, less than about 10%, less than about 9%, less than about 8%, less than about 7%, less than about 6%, less than about 5%, less than about 4%, less than about 3%, less than about 2%, less than about 1%, less than about 9‰, less than about 8‰, less than about 7‰, less than about 6‰, less than about 5‰, less than about 4‰, less than about 3‰, less than about 2‰, less than about 1‰, less than about 0.1‰, less than about 0.01‰, less than about 0.001‰, or even lower).

In the present application, the size of the fragment of the nucleic acid molecule derived from the microbial genome may be at least about 48kb (for example, it may be at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger).

In the present application, in the transfection mixture comprising the plasmid, the content of the nucleic acid molecule or fragments thereof with a size of at least about 48kb (for example, it may be at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger) may be less than about 10% of the total content of nucleic acid molecules in the transfection mixture (for example, it may be less than about 10%, less than about 9%, less than about 8%, less than about 7%, less than about 6%, less than about 5%, less than about 4%, less than about 3%, less than about 2%, less than about 1%, less than about 9%o, less than about 8‰, less than about 7‰, less than about 6‰, less than about 5‰, less than about 4‰, less than about 3‰, less than about 2‰, less than about 1‰, less than about 0.1‰, less than about 0.01‰, less than about 0.001‰, or even lower).

In the present application, the content of the nucleic acid molecule or fragment thereof with a size of at least about 48kb may be less than about 10%, less than about 9%, less than about 8%, less than about 7%, less than about 6%, less than about 5%, less than about 4%, less than about 3%, less than about 2%, less than about 1%, less than about 9‰, less than about 8‰, less than about 7‰, less than about 6‰, less than about 5‰, less than about 4‰, less than about 3‰, less than about 2‰, less than about 1‰, less than about 0.1 %o, less than about 0.01‰, less than about 0.001‰, or even lower of the total content of nucleic acid molecules in the transfection mixture. For example, the content of the nucleic acid molecule or fragment thereof with a size of at least 48kb may be less than about 2% of the total content of nucleic acid molecules in the transfection mixture; it may be less than about 5‰ of the total content of nucleic acid molecules in the transfection mixture; it may be less than about 1‰ of the total content of nucleic acid molecules in the transfection mixture.

In the present application, the nucleic acid molecule or fragment thereof with a size of at least about 48kb (e.g., may be at least about 50kb, at least about 100kb, at least about 150kb, at least about 200kb, at least about 250kb, at least about 300kb, at least about 350kb, at least about 400kb, at least about 450kb, at least about 500kb, at least about 600kb, at least about 700kb, at least about 800kb, at least about 900kb, at least about 1Mb, at least about 2Mb, at least about 3Mb, at least about 4Mb, at least about 5Mb, at least about 6Mb, at least about 7Mb, at least about 8Mb, at least about 9Mb, at least about 10Mb, at least about 20Mb, at least about 50Mb, at least about 100Mb, at least about 200Mb or larger) and the plasmid are present in different nucleic acid molecules. For example, the nucleic acid molecule or fragment thereof with a size of at least about 48kb is free in the transfection mixture (the transfection mixture may comprise plasmid). For example, the nucleic acid molecule or fragments thereof with a size of at least about 48kb is free from the plasmid. The freedom may be present in a separated manner without attachment to the plasmid.

In the present application, the plasmid (e.g., linear plasmid) may be treated (e.g., by deoxyribonuclease, e.g., by exonuclease, e.g., Exonuclease V) to make the content of the nucleic acid molecule or fragments thereof with a size of at least about 48kb (e.g., may be a linear nucleic acid molecule or fragments thereof) and/or the content of the nucleic acid molecule or fragment thereof derived from the microbial genome (e.g., may be a linear nucleic acid molecule or fragments thereof) meet the requiremnet of the present application. In the present application, the treatment may not affect nucleic acid molecules with a circular structure.

In the present application, the nucleic acid molecule or fragments thereof with a size of at least about 48kb may be derived from a microorganism. For example, the microorganism may be selected from one or more of the following group: bacteria, fungi, actinomycetes, mycoplasma, chlamydia, rickettsiae, and spirochetes.

In the present application, the microorganism may comprise Gram-negative bacteria. For example, the microorganism may be suitable for preparing a skeletal vector of the vector. For example, the microorganism may comprise *Escherichia coli.*

In the present application, the cell may comprise eukaryotic cell. The eukaryotic cell may comprise plant cell, fungal cell, and/or animal cell. Among them, the animal cell may comprise mammalian cell.

In the present application, the cell may comprise stem cell, immune cell, fibrocyte, and/or muscle cell. For example, the stem cell may comprise pluripotent stem cell. For example, the pluripotent stem cell may comprise embryonic stem cell (ESC). The embryonic stem cells can differentiate into three germ layers (e.g., ectoderm, mesoderm, and endoderm) and these three germ layers can ultimately form organs and tissues. The pluripotent stem cell may comprise epiblast stem cells (EpiSC). The pluripotent stem cell may also comprise induced pluripotent stem cell (e.g., iPS), which can be formed by dedifferentiation of mammalian somatic cells (e.g., skin cells from mouse tails) after the introduction of transcription factors (e.g., Oct4, SOX2, c-Myc, and Klf4).

In the present application, the stem cell may comprise hematopoietic stem cell and/or mesenchymal stem cell. The hematopoietic stem cell may differentiate into blood cell (e.g., blood cell of the bone marrow lineage and blood cell of the lymphoid lineage). The hematopoietic stem cell may have multipotency and self-renewal characteristics. The hematopoietic stem cell may differentiate into cells selected from the following group: monocytes, macrophages, neutrophils, basophils, eosinophils, erythrocytes, megakaryocytes, platelets, T cells, B cells, and NK cells.

The mesenchymal stem cell may be adult stem cell possessing self-renewal, multi-directional differentiation potential, and the ability to maintain their biological characteristics after large-scale *in vitro* expansion, derived from the mesoderm of the early embryonic development. The mesenchymal stem cell may express HLA-I class antigens. The mesenchymal stem cell may not express or may have low expression of HLA-II class antigens. The mesenchymal stem cell may have the ability to differentiate into adipocytes, osteocytes, and chondrocytes, and may also support the differentiation of hematopoietic stem cells into granulocytes, macrophages, and megakaryocytes. The mesenchymal stem cell may secrete cytokines, such as CSF-1, GM-CSF, G-CSF, IL-6, c-kitligand, and/or IL-3.

In the present application, the immune cell may comprise immune effector cell. For example, the immune effector cell may comprise lymphocytes (e.g., cytotoxic T cells, memory T cells), macrophages, dendritic cells, and NK cells. For example, the immune cell may be selected from the following group: T lymphocytes (e.g., may be activated T lymphocytes or non-activated T lymphocytes), B lymphocytes, NK cells, macrophages, dendritic cells, monocytes, granulocytes, and mast cells. For example, the immune cell may comprise primary T cell. The primary T cell may comprise T cell obtained from an individual (e.g., may be obtained from the peripheral blood T cell of the individual). The primary T cell may be different from T cells that have been maintained in culture for an extended period.

In the present application, the target molecule may comprise an antibody or antigen-binding fragment, chimeric antigen receptor (CAR), cytokine, and/or chemokine.

In the present application, the chimeric antigen receptor may target one or more targets.

In the present application, the chimeric antigen receptor may target CD19 and CD20.

In the present application, the chimeric antigen receptor may target CD19 and CD22.

In the present application, the antibody or antigen-binding fragment may comprise a bispecific antibody or antigen-binding fragment.

In the present application, the antibody or antigen-binding fragment may comprise a bispecific T cell engager (BiTE).

In the present application, the target molecule may be selected from the following group: antibodies (e.g., monoclonal antibodies, bispecific antibodies, and/or multispecific antibodies); Ig-type complete antibodies (e.g., IgG antibodies); antibody fragments (e.g., VHH, Fab, Fab', (Fab)₂, scFv); cytokines; chemokines; chimeric antigen receptors; and MHC complexes (e.g., HLA-A, HLA-G).

For example, the target molecule may comprise CART. For example, the target molecule may comprise CART and a cytokine (e.g., IL-15). For example, the target molecule may comprise CART-BiTE.

In the present application, the nucleic acid molecule encoding the target molecule and/or the nucleic acid molecule encoding the screening marker molecule may be natural or may be engineered genes.

In the present application, the plasmid may comprise homology arms that are homologous to the ends of the nucleic acid molecule encoding the target molecule and/or the nucleic acid molecule encoding the screening marker molecule or fragments thereof. For example, the 5' end and/or 3' end of the nucleic acid molecule or fragment thereof may have a nucleic acid sequence with at least about 80%, at least about 85%, at least about 90%, at least about 91%, at least about 92%, at least about 93%, at least about 94%, at least about 95%, at least about 96%, at least about 97%, at least about 98%, at least about 99%, or 100% identity to the 5' end and/or 3' end of the nucleic acid sequence to be knocked in the chromosomal DNA of the cell to be transfected. For example, the homology arm may be 100-1000 bp in size. For example, the homology arm may be about 150 bp-1000 bp, may be about 200 bp-1000 bp, may be about 300 bp-1000 bp, may be about 400 bp-1000 bp, or may be about 800 bp-1000 bp.

In the present application, the screening marker molecule may comprise CD34 and/or EGFRT variant. In the present application, the screening marker molecule may also comprise an antibiotic.

In the present application, the expression and/or function of the screening marker molecule does not affect the expression and/or function of the target molecule.

In the present application, the type of screening marker molecule may correspond to the type of cell and/or target molecule. For example, the cell may be an immune cell, and the screening marker molecule may be CD34 and/or EGFRT variant. For example, the cell may be a CAR-T cell, and the screening marker molecule may be CD34.

In the present application, the method may comprise the following steps: contacting the cell expressing the target molecule and the screening marker molecule with the screening material.

In the present application, the method may also comprise the following steps: collecting the cells bound to the screening material.

In the present application, the method may also comprise the following steps: separating the cell bound to the screening material from the screening material.

In the present application, the screening marker molecule may comprise a structural domain capable of binding to the screening material. In the present application, cells transfected with the desired target molecule and screening marker molecule can be collected (e.g., separated from the screening material) and/or enriched by the binding of the screening molecule to the screening material. For example, the screening marker molecule may be CD34, and cells expressing CD34 in the method of the present application (e.g., CAR-T cells) may be contacted with microbeads containing CD34 antibodies. Due to the interaction between CD34 and CD34 antibodies, cells expressing CD34 bind to the microbeads. Cells bound to the CD34-expressing microbeads are collected. Furthermore, the collected cells can be separated from the microbeads. In this way, cells expressing the target molecule and the screening marker molecule (i.e., positive cells) can be obtained. Therefore, the method of the present application can improve the positive rate of cell transfection (e.g., non-viral transfection).

In the present application, the screening material may comprise magnetic beads. For example, the screening material may comprise micro-magnetic beads.

In the present application, the method may also comprise the following step: treating the plasmid with deoxyribonuclease (DNase).

For example, after the DNase treatment, in the transfection mixture containing the plasmid, the content of nucleic acid molecule or fragment thereof derived from the microbial genome may be less than about 10% of the total content of nucleic acids in the transfection mixture. Furthermore, for example, after the DNase treatment, in the transfection mixture containing the plasmid, the content of nucleic acid molecule or fragment thereof with a size of at least about 48 kb may be less than about 10% of the total content of nucleic acids in the transfection mixture.

In the present application, the method of the present application may comprise the following step: reducing the content of the nucleic acid molecule or fragment thereof in the transfection mixture. In the present application, the reduction may refer to reducing the content of the nucleic acid molecule or fragment thereof in the transfection mixture to meet the requirements of the method of the present application.

In the present application, the reduction may comprise purifying the transfection mixture. That is, the content of the nucleic acid molecule or fragment thereof in the transfection mixture may be reduced to meet the requirements of the method of the present application through the purification. In the present application, the purification may remove the nucleic acid molecule or fragment thereof with a size of at least about 48 kb; and/or, remove the nucleic acid molecule or fragment thereof derived from the microbial genome. For example, the purification may comprise using methods well known to those skilled in the art for purifying DNA to reduce the content of the nucleic acid molecule or fragment thereof in the transfection mixture. In the present application, the purification may not affect the integrity and/or activity of the plasmid DNA. In the present application, the reduction may comprise purifying the transfection mixture using a reagent selected from the following group: DNAase, SDS, TX-100, CTAB, and cesium chloride-ethidium bromide. For example, the reduction may use DNAase. For example, the use of DNAase may reduce the content of nucleic acid molecule or fragment thereof with a size of at least about 48 kb in the transfection mixture to meet the requirements of the method of the present application; and/or, reduce the content of nucleic acid molecule or fragment thereof derived from the microbial genome to meet the requirements of the method of the present application. For example, the DNAase may not affect the integrity and/or activity of the circular plasmid DNA.

In the present application, the reduction may comprise using a method selected from the following group: DNA synthesis of the plasmid according to the present application and HPLC detection of the transfection mixture according to the present application. For example, direct DNA synthesis can be performed based on the nucleotide sequence of the plasmid, which can result in a plasmid containing only the nucleotide sequence of the plasmid. For example, the transfection mixture of the present application can be detected by HPLC, and based on the HPLC detection results, the characteristic peak corresponding to the plasmid can be selected, and the component corresponding to the characteristic peak can be collected, which can result in a plasmid containing only the nucleotide sequence of the plasmid.

In the present application, the method of the present application can significantly improve the transfection efficiency and/or positive rate of cells, thereby achieving the preparation of modified cells for cell therapy using non-viral transfection methods. The modified cells obtained by the method of the present application can have a high cell viability and/or sustained proliferative capacity (for example, the cells can maintain a high proliferative capacity for a detection time range after transfection (such as electroporation) (for example, at least 9 days)). The modified cells obtained by the method of the present application can have a high cellular function (for example, they can have the ability to effectively kill tumor cells). The method of the present application can be used to prepare modified cells required for cell therapy (such as CAR-T cells). The method of the present application can be used to prepare CAR-T cells and/or CAR-T-BiTE cells based on primary T cells using non-viral transfection methods (such as plasmid transfection).

In another aspect, the present application provides a cell and/or cell line obtained by the method of the present application.

In another aspect, the present application provides a modified immune cell that simultaneously expresses a target molecule and a screening marker molecule.

In the present application, the immune cell may be selected from the following group: unactivated or activated T lymphocytes, B lymphocytes, NK cells, macrophages, dendritic cells, monocytes, granulocytes, and mast cells.

In the present application, the target molecule may comprise an antibody or antigen-binding fragment, a chimeric antigen receptor (CAR), a cytokine, and/or a chemokine.

In the present application, the chimeric antigen receptor may target one or more targets.

In the present application, the chimeric antigen receptor may target CD19 and CD20.

In the present application, the chimeric antigen receptor may target CD19 and CD22.

In the present application, the antibody or antigen-binding fragment may comprise a bispecific antibody or antigen-binding fragment.

In the present application, the antibody or antigen-binding fragment may comprise a bispecific T cell engager (BiTE).

In the present application, the screening marker molecule may comprise a structural domain capable of binding to a screening material.

In the present application, the screening material may comprise magnetic beads.

In the present application, the screening marker molecule may comprise CD34 and/or EGFRT variant.

In the present application, the type of screening marker molecule may be selected based on the type of the cell and/or the target molecule.

In the present application, the cell may be an immune cell, and the screening marker molecule may be CD34 and/or EGFRT variant.

Without being limited by any theory, the examples below are merely for illustrating various technical solutions of the present application and are not intended to limit the scope of the present application.

### Examples

### Example 1 Characterization of modified cells obtained by non-viral transfection

### (1.1) Detection of the content of nucleic acid molecules derived from microbial genomes in plasmids

Using the *E. coli* DNA Quantitation Kit (Cat# 4458435, Thermo Fisher), the content of nucleic acid molecule or fragment thereof derived from microbial genomes (mainly E. coli genomic DNA) in commercially available plasmids (referred to as A source (GenScript) plasmids and B source (Sino Biological) plasmids according to different purchase sources) was quantified by qPCR according to the method described in the kit instructions. qPCR was performed using a CF96 Tough qPCR instrument (purchased from Bio-Rad).

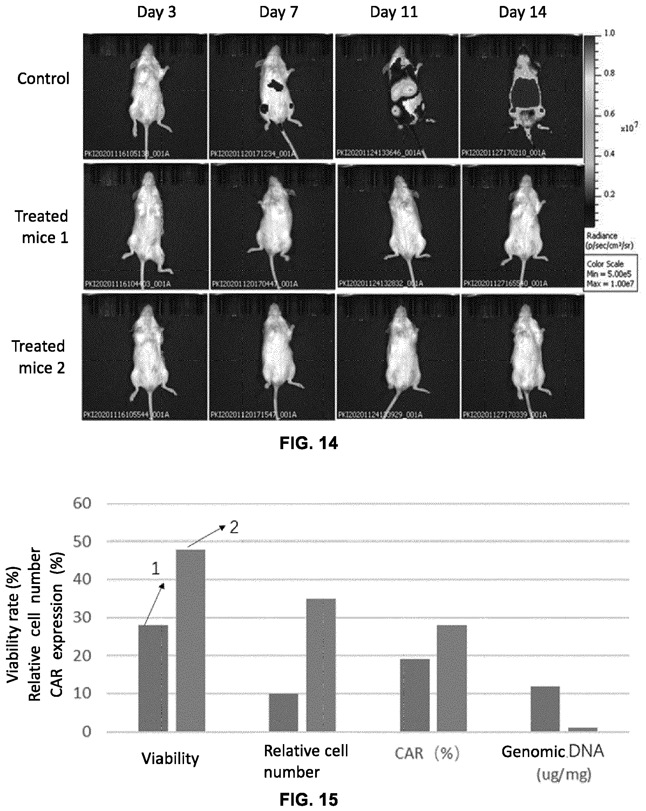

The results of the measurement are shown in FIG. 15. The results in FIG. 15 show that there are significant differences in the content of nucleic acid molecule or fragment thereof derived from the microbial genome in these plasmids. Among them, the results of plasmid A are shown in column 1; the results of plasmid B are shown in column 2. In plasmid A, the content of nucleic acid molecule derived from the microbial genome accounts for 12µg/mg of the total nucleic acid molecule content; in plasmid B, the content of nucleic acid molecules derived from the microbial genome accounts for less than 1 |ig/mg of the total nucleic acid molecule content.

Plasmids A and B each comprise nucleic acid molecules capable of expressing CD 19 CAR and CD34. The nucleotide sequence of the nucleic acid molecule encoding CD34 is shown in SEQ ID NO.4. The nucleotide sequence of the nucleic acid molecule encoding CD19 CAR is shown in SEQ ID NO.5.

The plasmids are referred to as CD19 CAR-CD34-A and CD19 CAR-CD34-B, respectively, and were electroporated into human T cells that have been activated with Dynal beads (using Dynal beads and collected human T cells in X-vivo 15+20U/ml culture medium at a 1:1 ratio) for two days. The cell viability, relative cell number from cell proliferation, and CAR expression capability were measured on day 1 or day 4 after electroporation. The results are shown in FIG. 15, where the results of plasmid A are shown in column 1; the results of plasmid B are shown in column 2.

The results in FIG. 15 indicate that when the content of nucleic acid molecules derived from the microbial genome in the plasmids is reduced, the cell viability, cell proliferation, and/or expression capability (e.g., expression of CD19 CAR) can be significantly improved after plasmid transfection.

### (1.2) Preparation of plasmids capable of expressing GPC3 CAR and CD34. The nucleotide sequence of the nucleic acid molecule encoding CD34 is shown in SEQ ID NO.4. The nucleotide sequence of the nucleic acid molecule encoding GPC3 CAR is shown in SEQ ID NO.3.

Human T cells are activated with Dynal beads in X-vivo15+20U/ml culture medium at a 1:1 ratio.

The prepared plasmids were electroporated into human T cells that have been activated with Dynal beads for two days. The nucleic acid molecules encoding GPC3 CAR and CD34 were knocked into the genome of the human T cells and may be regulated for expression by endogenous promoters. The resulting cells are named GPC3 CAR-CD34 T cells.

### (1) Cell Viability

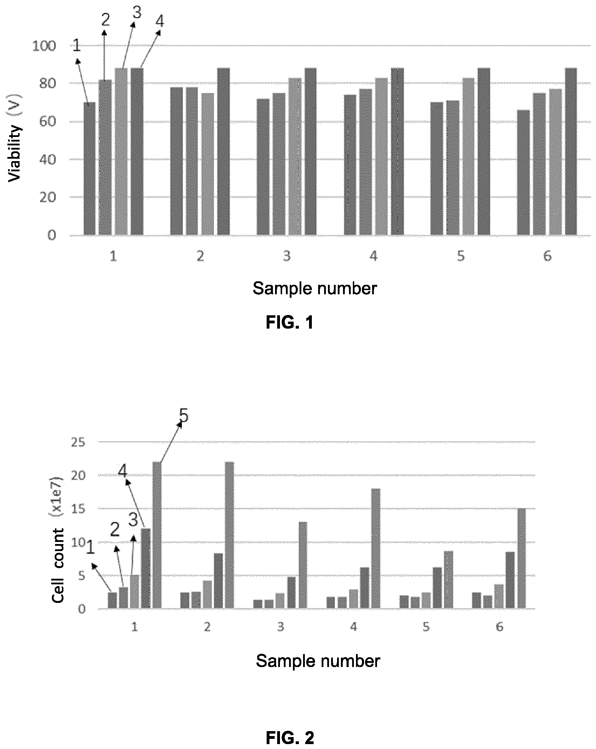

FIG. 1 shows the cell viability of human T cells for different samples, on Days 1-4 after electroporation of the plasmids. Cell viability is obtained by counting with NC-200. The results 1-4 from left to right for each sample represent the results of day 1, day 2, day 3 and day 4 after electroporation, respectively. Thus, modifying the cells does not affect their cell viability.

### (2) Cell Proliferation

FIG. 2 shows the cell counts of human T cells for different samples, on days 0-4 after electroporation of the plasmids. Cell viability is obtained by counting with NC-200. The results 1-5 from left to right for each sample represent the results of day 0, day 1, day 2, day 3 and day 4 after electroporation, respectively. Thus, modifying the cells does not affect their cell proliferation.

### (3) Expression Capability

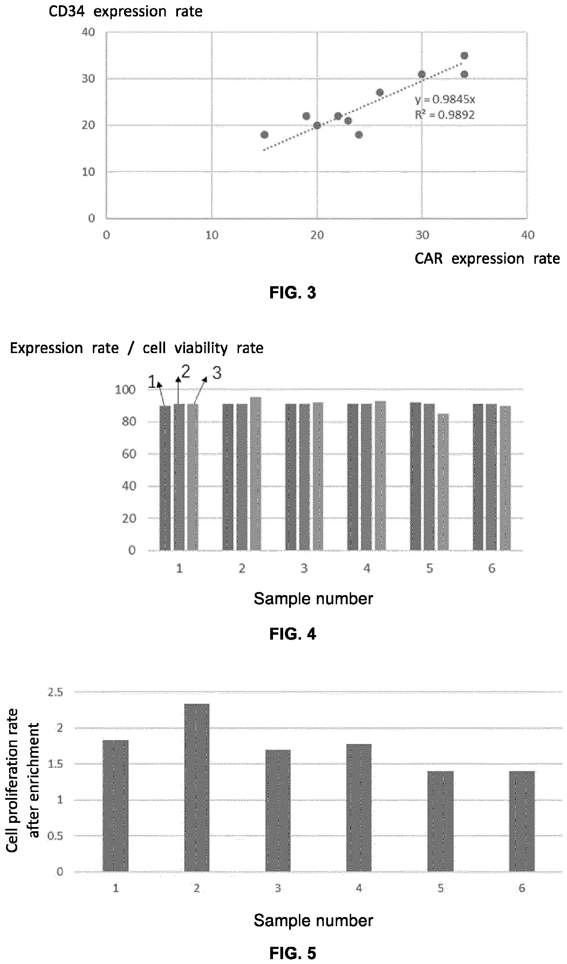

The expression rates of GPC3 CAR and CD34 in GPC3 CAR-CD34 T cells were separately counted. The results are shown in FIG. 3. It can be seen that there is a linear relationship between the expression of GPC3 CAR and CD34 in GPC3 CAR-CD34 T cells.

### Example 2 Characteristics of Enriched Modified Cells

The enrichment of GPC3 CAR-CD34 T cells prepared in Example 1 was performed using the enrichment steps provided by the CD34 MicroBeads Kit UltraPure human (Order NO. 130-100-453, Miltenyi Biotech), which specifically binds CD34-expressing cells and CD34 antibody-containing magnetic beads. Enriched GPC3 CAR-CD34 T cells were obtained.

### (1) Expression Capability and Cell Viability

The expression rates of GPC3 CAR and CD34 in enriched GPC3 CAR-CD34 T cells were separately counted. The results are shown in FIG. 4. The results (1-3) from left to right for each sample represent the expression rates of GPC3 CAR, CD34, and the cell viability of enriched GPC3 CAR-CD34 T cells, respectively. It can be seen that the enrichment operation does not affect the expression of GPC3 CAR and CD34, and it does not affect the cell viability of GPC3 CAR-CD34 T cells.

### (2) Cell Proliferation

The cell doubling rate of enriched GPC3 CAR-CD34 T cells in each sample on day 4 after electroporation with the plasmid of Example 1 was calculated. The doubling rate = (number of enriched GPC3 CAR-CD34 T cells on day 4 after electroporation) / (number of T cells to be electroporated on day 0). The results are shown in FIG. 5.

The results in FIG. 5 indicate that enriched GPC3 CAR-CD34 T cells still have high cell proliferation capability after electroporation.

### (3) Positive Rate

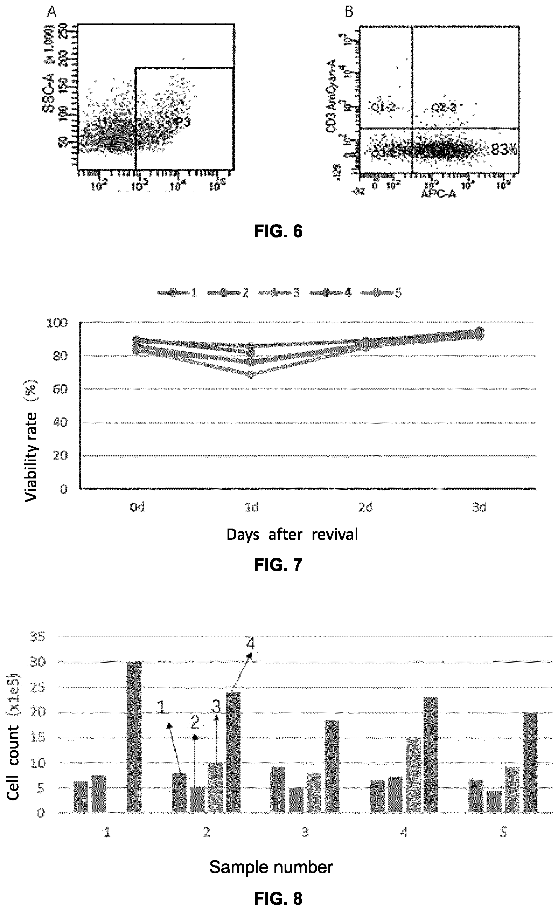

Using flow cytometry, the positive rates of GPC3 CAR expression in GPC3 CAR-CD34 T cells prepared in Example 1 and enriched GPC3 CAR-CD34 T cells prepared in Example 2 were separately counted. The results are shown in Figures 6A-6B, respectively. The results show that the positive rate can be increased from about 20% before enrichment to 83% after enrichment. It can be seen that the enriched modified cells can effectively improve the positive rate of expression.

### Example 3 Recovery Ability of Modified Cells

The GPC3 CAR-CD34 T cells prepared in Example 1 from 5 samples on day 4 after electroporation were enriched using magnetic beads containing CD34 antibody (MicroBead), and stored after enrichment. The specific steps for cryopreservation are as follows: centrifuge cells (1000RPM, 5min), remove the supernatant. Centrifuge again to remove any adhering culture supernatant from the tube wall. Add 4°C CryoStor CS10 freezing medium (Stemcell Technologies, Canada), place in a CoolCell freezing box, leave at -80°C overnight, and then transfer to liquid nitrogen.

### (1) Viability

The cryopreserved cells were revived and the viability of GPC3 CAR-CD34 T cells on different days after revival was detected. The results are shown in FIG. 7. The results of FIG. 7 indicate that the modified cells of the present application have good recovery ability after cryopreservation.

### (2) Cell proliferation

The cryopreserved cells were revived and the cell count of GPC3 CAR-CD34 T cells on different days after revival was detected. The results are shown in FIG. 8. The results 1-4 from left to right for each sample represent the cell counts on day 0, day 1, day 2, and day 3 after revival, respectively. The results of FIG. 8 indicate that the modified cells of the present application still have good proliferation ability after recovery from cryopreservation.

### (3) Activation ability of target cells

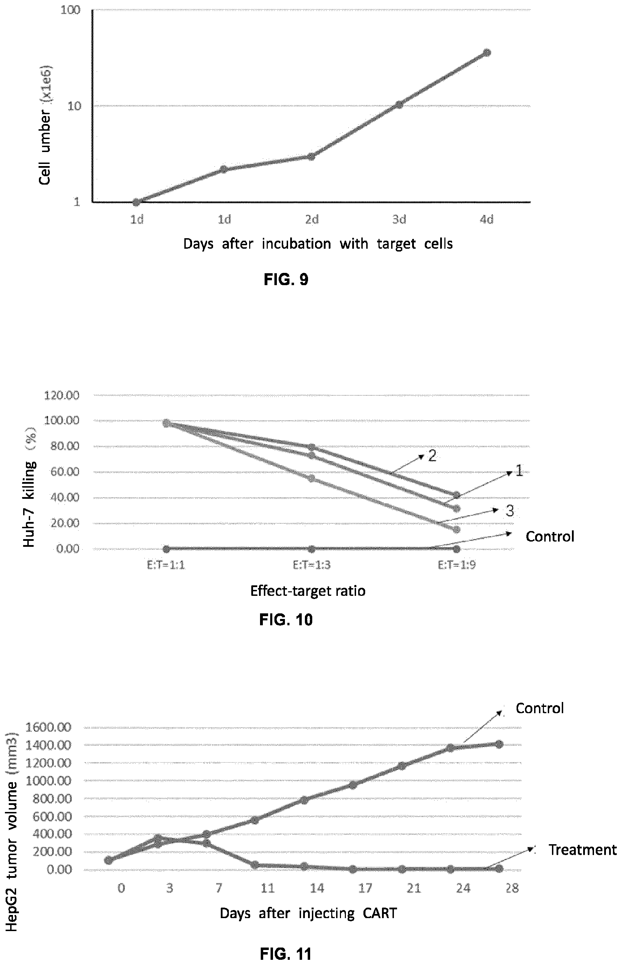

The cryopreserved cells were revived, and on day 3 after revival, the revived GPC3 CAR-CD34 T cells were mixed with Huh-7 target cells at a 1:1 effector-target ratio, and the cell count of GPC3 CAR-CD34 T cells after incubation was detected. The results are shown in FIG. 9. The results of FIG. 9 indicate that the modified cells of the present application still have the ability to expand when activated by target cells after recovery from cryopreservation.

### Example 4 Killing ability of Modified Cells against Tumors

### (1) In vitro killing ability

The enriched GPC3 CAR-CD34 T cells prepared in Example 2 were mixed with Huh-7 target cells at different effector-target ratios *in vitro,* and the killing percentage (%) of Huh-7 was detedced. The results are shown in FIG. 10. The control refers to T cells that have not been transfected with the plasmid of Example 1. Numbers 1-3 represent the conditions of enriched GPC3 CAR-CD34 T cells from different sample sources. The results of FIG. 11 indicate that the modified cells of the present application still have killing ability against target cells after recovery from cryopreservation.

### (2) In vivo killing ability

The GPC3 CAR-CD34 T cells prepared in Example 1 were enriched with CD34 antibody-containing microbeads (MicroBead) on day 4 after electroporation and cryopreserved after enrichment.

HepG2 cells were injected into mice (NCG mice, purchased from Gempharmatech Co., Ltd.) to obtain a subcutaneous HepG2-bearing mouse model.

When the tumor volume reaches approximately 100³mm, the revived cells were injected at a dose of 1e7 into the obtained mouse model via tail vein (4 mice per treatment). The average tumor volume was detected in the mice after treatment. The results are shown in FIG. 11. The control refers to T cells transfected with CD 19 CAR (the nucleotide sequence of the nucleic acid molecule encoding CD19 CAR is shown in SEQ ID NO. 5).

The results of FIG. 11 indicate that the modified cells of the present application still have specific killing ability against tumors *in vivo* after recovery from cryopreservation.

### Example 5 Killing ability of modified cells against tumors

A plasmid capable of expressing CD19 CAR and CD34 was prepared. The nucleotide sequence of the nucleic acid molecule encoding CD19 CAR is shown in SEQ ID NO. 5. The nucleotide sequence of the nucleic acid molecule encoding CD34 is shown in SEQ ID NO. 4. The nucleotide sequence of the nucleic acid molecule encoding GPC3 CAR-CD34 knocked into the TRAC locus is shown in SEQ ID NO. 1.

Human T cells collected with Dynal beads were activated at a 1:1 ratio in X-vivo15+20U/ml culture medium.

The prepared plasmid was electroporated into human T cells activated with Dynal beads for two days, and the nucleic acid molecules encoding CD19 CAR and CD34 were knocked into the human T cell genome and can be regulated by endogenous promoters for expression. The resulting cells are named CD19 CAR-CD34 T cells.

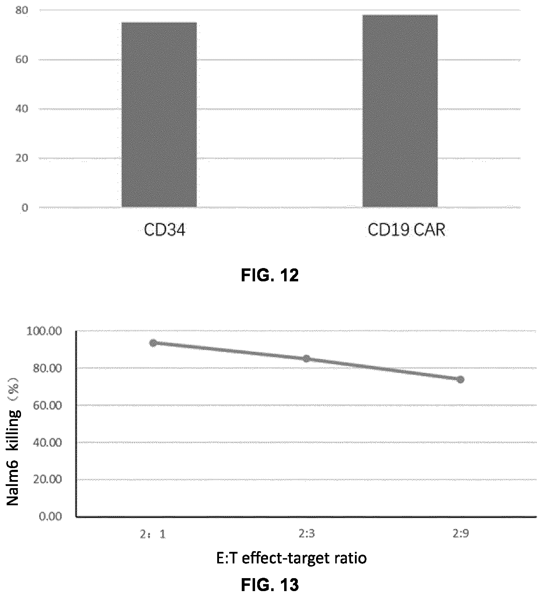

On day 4 after electroporation, CD19 CAR-CD34 T cells are enriched using MicroBeads containing CD34 antibody. The expression rates of CD19 CAR and CD34 in the enriched CD19 CAR-CD34 T cells are counted separately. The results are shown in FIG. 12. The results indicate that the enriched CD19 CAR-CD34 T cells can normally express CD19 CAR and CD34.

### (1) In vitro killing ability

The enriched CD19 CAR-CD34 T cells were mixed with Nalm6 target cells *in vitro* at different effector-to-target ratios, and the killing percentage (%) of Nalm6 was detected. The results are shown in FIG. 13. The results in FIG. 13 indicate that the enriched CD19 CAR-CD34 T cells have killing ability against target cells *in vitro.*

### (2) In vivo killing ability

Fluorescent Nalm6 cells (Nalm-6-Luc, purchased from PharmaLegacy, Shanghai) were intravenously injected into mice (NCG mice, purchased from Gempharmatech Co., Ltd.) to obtain a mouse model of blood cancer Nalm6.

The enriched CD19 CAR-CD34 T cells were injected into the resulting mouse model at a dose of 1e7 via tail vein injection (4 mice for each treatment). The growth and killing of the tumor were detected by the fluorescence value of Nalm-6-Luc (Lumina XRMS, PerkinElmer). The results are shown in FIG. 14. The control refers to T cells transfected with GPC3 CAR (the nucleotide sequence of the nucleic acid molecule encoding GPC3 CAR is shown in SEQ ID NO. 3).

The results in FIG. 14 indicate that the modified cells of the present application still have the ability to kill tumors *in vivo* after recovery from cryopreservation.

The above detailed description is provided for explanation and example purposes and is not intended to limit the scope of the appended claims. Variations of the embodiments listed in the present application are apparent to those skilled in the art and are reserved within the scope of the appended claims and their equivalents.

## Claims

1. A method for modifying a cell, comprising the following step: introducing a target molecule and/or a screening marker molecule into a cell by non-viral transfection, so that the cell expresses the target molecule and the screening marker molecule.

2. A method for increasing positive rate of target molecule transfection, comprising the following steps: introducing a target molecule and/or a screening marker molecule into a cell by non-viral transfection, so that the cell expresses the target molecule and the screening marker molecule.

3. The method according to any one of claims 1-2, wherein the cell simultaneously expresses the target molecule and the screening marker molecule.

4. The method according to any one of claims 1-3, wherein the transfection comprises transient transfection.

5. The method according to any one of claims 1-4, wherein the transfection comprises electroporation.

6. The method according to any one of claims 1-5, wherein the transfection comprises stable transfection.

7. The cell according to any one of claims 1-6, wherein the target molecule and/or the screening marker molecule are integrated into cell genome.

8. The method according to any one of claims 1-7, wherein the transfection comprises using a transposon system and/or using gene editing method for knock-in.

9. The method according to claim 8, wherein the gene editing method is selected from the following group consisting of: CRISPR/Cas system, RNA editing system ADAR, RNA-guided endonucleases, zinc finger nucleases, Mega-TAL nucleases, TALENs, and Meganucleases.

10. The method according to any one of claims 1-9, wherein the non-viral transfection uses linear or circular nucleic acid molecule having an encoding region.

11. The method according to claim 10, wherein the circular nucleic acid molecule comprises a circular plasmid or a supercoiled plasmid.

12. The method according to claim 11, wherein the plasmid comprises nucleic acid molecule encoding the target molecule and/or a nucleic acid molecule encoding the screening marker molecule.

13. The method according to claims 11-12, wherein the plasmid acts as a donor plasmid in a knock-in process via gene editing.

14. The method according to any one of claims 10-13, wherein the nucleic acid molecule comprises ssDNA and/or dsDNA.

15. The method according to any one of claims 13-14, wherein the donor plasmid comprises homology arms.

16. The method according to any one of claims 11-15, wherein the plasmid comprises a plasmid produced by bacteria.

17. The method according to any one of claims 11-16, wherein the plasmid comprises nanoplasmid and/or minicircle DNA vector.

18. The method according to any one of claims 11-17, wherein in a transfection mixture containing the plasmid, the content of nucleic acid molecule or fragment thereof derived from a microbial genome is less than about 10% of the total content of nucleic acid molecule in the transfection mixture.

19. The method according to claim 18, wherein in the transfection mixture comprising the plasmid, the content of nucleic acid molecule or fragment thereof derived from a microbial genome is less than about 2% of the total content of nucleic acid molecules in the transfection mixture.

20. The method according to any one of claims 18-19, wherein in the transfection mixture comprising the plasmid, the content of nucleic acid molecule or fragment thereof derived from a microbial genome is less than about 5‰ of the total content of nucleic acid molecules in the transfection mixture.

21. The method according to any one of claims 18-20, wherein in the transfection mixture comprising the plasmid, the content of nucleic acid molecule or fragment thereof derived from a microbial genome is less than about 1‰ of the total content of nucleic acid molecules in the transfection mixture.

22. The method according to any one of claims 11-21, wherein in the transfection mixture comprising the plasmid, the content of nucleic acid molecule or fragment thereof with a size of at least about 48 kb is less than about 10% of the total content of nucleic acid molecules in the transfection mixture.

23. The method according to claim 22, wherein in the transfection mixture comprising the plasmid, the content of nucleic acid molecule or fragment thereof with a size of at least about 48 kb is less than about 2% of the total content of nucleic acid molecules in the transfection mixture.

24. The method according to any one of claims 22-23, wherein in the transfection mixture comprising the plasmid, the content of nucleic acid molecule or fragment thereof with a size of at least about 48 kb is less than about 5‰ of the total content of nucleic acid molecules in the transfection mixture.

25. The method according to any one of claims 22-24, wherein in the transfection mixture comprising the plasmid, the content of nucleic acid molecule or fragment thereof with a size of at least about 48 kb is less than about 1‰ of the total content of nucleic acid molecules in the transfection mixture.

26. The method according to any one of claims 22-25, wherein the size of the nucleic acid molecule or fragment thereof with a size of at least about 48 kb is at least about 1 Mb.

27. The method according to any one of claims 22-26, wherein the size of the nucleic acid molecule or fragment thereof with a size of at least about 48 kb is at least about 10 Mb.

28. The method according to any one of claims 22-27, wherein the nucleic acid molecule or fragment thereof with a size of at least about 48 kb is derived from a microorganism.

29. The method according to claim 28, wherein the microorganism is selected from the following group consisting of: bacteria, fungi, actinomycetes, mycoplasmas, chlamydiae, rickettsiae, and spirochetes.

30. The method according to any one of claims 28-29, wherein the microorganism comprises Gram-negative bacteria.

31. The method according to any one of claims 28-30, wherein the microorganism comprises *Escherichia coli.*

32. The method according to any one of claims 11-31, comprising a step of treating the plasmid with deoxyribonuclease (DNase).

33. The method according to claim 32, wherein the DNase is capable of non-specifically cleaving linear DNA.

34. The method according to any one of claims 32-33, wherein the DNase is an exonuclease.

35. The method according to any one of claims 32-34, wherein the treatment comprises contacting the transfection mixture with the DNase in the presence of Mg²⁺ and Ca²⁺.

36. The method according to any one of claims 32-35, wherein after the treatment by the DNase, in the transfection mixture comprising the plasmid, the content of nucleic acid molecule or fragment thereof derived from a microbial genome is less than about 10% of the total content of nucleic acid molecules in the transfection mixture.

37. The method according to any one of claims 32-35, wherein after the treatment by the DNase, in the transfection mixture comprising the plasmid, the content of nucleic acid molecule or fragment thereof with a size of at least about 48 kb is less than about 10% of the total content of nucleic acid molecules in the transfection mixture.

38. The method according to any one of claims 1-37, wherein the cell comprises eukaryotic cell.

39. The method according to any one of claims 1-38, wherein the cell comprises immune cell, stem cell, fibrocyte, and/or muscle cell.

40. The method according to claim 39, wherein the stem cell comprises pluripotent stem cell.

41. The method according to any one of claims 39-40, wherein the stem cell comprises hematopoietic stem cell and/or mesenchymal stem cell.

42. The method according to any one of claims 1-41, wherein the immune cell is selected from the group consisting of: unactivated or activated T lymphocyte, B lymphocyte, NK cell, macrophage, dendritic cell, monocyte, granulocyte, and mast cell.

43. The method according to any one of claims 39-42, wherein the immune cell comprises primary T cell.

44. The method according to any one of claims 1-43, wherein the target molecule comprises antibody or antigen-binding fragment, chimeric antigen receptor (CAR), cytokine, and/or chemokine.

45. The method according to claim 44, wherein the chimeric antigen receptor targets one or more targets.

46. The method according to any one of claims 44-45, wherein the chimeric antigen receptor targets CD19 and CD20.

47. The method according to any one of claims 44-45, wherein the chimeric antigen receptor targets CD19 and CD22.

48. The method according to any one of claims 44-47, wherein the antibody or antigen-binding fragment comprises bispecific antibody or antigen-binding fragment.

49. The method according to any one of claims 44-48, wherein the antibody or antigen-binding fragment comprises bispecific T cell engager (BiTE).

50. The method according to any one of claims 1-49, wherein the screening marker molecule comprises a structural domain capable of binding to a screening material.

51. The method according to claim 50, wherein the screening material comprises magnetic bead.

52. The method according to any one of claims 1-51, wherein the screening marker molecule comprises CD34 and/or EGFRT (epidermal growth factor receptor truncated) variant.

53. The method according to any one of claims 1-52, wherein the type of the screening marker molecule corresponds to the type of the cell and/or the type of the target molecule.

54. The method according to any one of claims 1-53, wherein the cell is immune cell and the screening marker molecule is CD34 and/or EGFRT variant.

55. The method according to any one of claims 50-54, comprising the following step: contacting the cell expressing the target molecule and the screening marker molecule with the screening material.

56. The method according to claim 55, further comprising the following step: collecting the cells bound to the screening material.

57. The method according to claim 56, further comprising the following step: separating the cells bound to the screening material from the screening material.

58. A cell and/or a cell line prepared by a method according to any one of claims 1-57.

59. A modified immune cell simultaneously expressing a target molecule and a screening marker molecule.

60. The immune cell according to claim 59, wherein the immune cell is selected from the group consisting of: unactivated or activated T lymphocyte, B lymphocyte, NK cells macrophage, dendritic cell, monocyte, granulocyte, and mast cell.

61. The immune cell according to any one of claims 59-60, wherein the target molecule comprises antibody or antigen-binding fragment, chimeric antigen receptor (CAR), cytokine, and/or chemokine.

62. The immune cell according to claim 61, wherein the chimeric antigen receptor targets one or more targets.

63. The immune cell according to any one of claims 61-62, wherein the chimeric antigen receptor targets CD19 and CD20.

64. The immune cell according to any one of claims 61-63, wherein the chimeric antigen receptor targets CD19 and CD22.

65. The immune cell according to any one of claims 61-64, wherein the antibody or antigen-binding fragment comprises bispecific antibody or antigen-binding fragment.

66. The immune cell according to any one of claims 61-65, wherein the antibody or antigen-binding fragment comprises bispecific T cell engager (BiTE).

67. The immune cell according to any one of claims 59-66, wherein the screening marker molecule comprises structural domain capable of binding to a screening material.

68. The immune cell according to claim 67, wherein the screening material comprises magnetic bead.

69. The immune cell according to any one of claims 59-68, wherein the screening marker molecule comprises CD34 and/or EGFRT variant.

70. The immune cell according to any one of claims 59-69, wherein the type of the screening marker molecule is selected based on the type of the cell and/or the type of the target molecule.

71. The immune cell according to any one of claims 59-70, wherein the cell is immune cell and the screening marker molecule is CD34 and/or EGFRT variant.
